# FASCICULE DE BREVET EUROPEEN

(11) **EP 4 164 763 B1**
(45) Date de publication et mention de la délivrance du brevet: **03.07.2024**
(21) Numéro de dépôt: 21731188.5
(22) Date de dépôt: 11.06.2021
(51) Int. Cl.: B01D 11/02

(54) **DISPOSITIF D'EXTRACTION SOLIDE/LIQUIDE PAR IRRADIATION ULTRASONORE RADIALE, ET PROCÉDÉ D'EXTRACTION ASSOCIÉ**
VORRICHTUNG ZUR FEST-/FLÜSSIGEXTRAKTION DURCH RADIALE ULTRASCHALLBESTRAHLUNG UND ZUGEHÖRIGES EXTRAKTIONSVERFAHREN
DEVICE FOR SOLID/LIQUID EXTRACTION BY RADIAL ULTRASONIC IRRADIATION, AND ASSOCIATED EXTRACTION METHOD

(30) Priorité: 12.06.2020 FR 2006171
(43) Date de publication de la demande: 19.04.2023
(73) Titulaire: Université Savoie Mont Blanc, 73000 Chambéry (FR)
(72) Inventeur: CHATEL, Grégory, 01300 BRENS (FR); DRAYE, Micheline, 73000 CHAMBERY (FR); DUWALD, Romain, 73000 CHAMBERY (FR); FANGET, Philippe, 73240 SAINT GENIX LES VILLAGES (FR); PIOT, Christine, 73230 BARBY (FR)
(74) Mandataire: Lavoix
(86) Numéro de dépôt international: PCT/EP2021/065820
(87) Numéro de publication internationale: WO 2021/250251

(56) Documents cités:
- WO-A2-2011/136514
- CN-B- 104 784 964
- CN-Y- 2 401 245
- DE-B- 1 113 863
- FR-A1- 3 066 703

## Description

La présente invention concerne un dispositif d'extraction solide/liquide.

Parmi les dispositifs conventionnels d'extraction solide/liquide, en particulier pour obtenir des molécules d'intérêt à partir de broyat végétal, il est courant d'employer des appareils à ultrasons. En effet, les dispositifs optimisés impliquant les ultrasons peuvent permettre de limiter la consommation énergétique liée à un chauffage prolongé et éliminer l'utilisation de solvants organiques potentiellement toxiques. Ils permettent également d'opérer à température ambiante et donc de limiter la dégradation de certaines molécules organiques sensibles.

A cet effet, des bacs à ultrasons sont couramment utilisés en industrie permettant de traiter d'importants volumes et de procéder par lots. Néanmoins, ces dispositifs non optimisés présentent de nombreux inconvénients pour l'extraction tels que l'impossibilité de contrôler l'élévation de la température engendrée par les ultrasons pouvant conduire à la dégradation de certaines molécules thermosensibles.

De plus, l'extraction en bacs à ultrasons ne peut aujourd'hui être réalisée que par lots. Ceci requiert de vider intégralement les bacs après chaque lot traité, de les nettoyer et de procéder à une filtration du milieu sur un équipement annexe. Il est donc obligatoire d'ajouter des étapes de transfert et de transport du mélange, entrainant des pertes de matière et des temps de manutention importants.

Des exemples de dispositifs courants d'extraction sont à titre d'exemples non limitatifs, décrits dans les documents DE 1113863, CN 104784964 B, CN 2401245 Y et FR 3066703 A1.

Un des buts de l'invention est de proposer un dispositif d'extraction solide/liquide en continu peu encombrant, permettant l'extraction rapide et efficace de molécules d'intérêt à partir de matière solide mise en suspension dans un solvant liquide, tout en maintenant la fluidité et l'homogénéité de la suspension et permettant de s'astreindre d'une étape de filtration par la suite.

A cet effet, l'invention a pour objet un dispositif d'extraction solide/liquide en continu comprenant au moins, d'amont en aval :
un mélangeur comprenant une entrée et une sortie, un serpentin d'extraction comprenant une entrée et une sortie, l'entrée du serpentin étant connectée à la sortie du mélangeur, et un séparateur de phases connecté à la sortie du serpentin,
caractérisé en ce qu'il comprend une sonde ultrasonore à irradiation radiale placée au centre du serpentin.

Le dispositif d'extraction solide/liquide selon l'invention peut comprendre l'une ou plusieurs des caractéristiques suivantes, prise(s) isolément ou suivant toute combinaison technique possible :
- le serpentin est formé d'un tube s'enroulant en hélice autour d'un axe pour former des spires, et dans lequel la sonde ultrasonore s'étend principalement suivant une direction d'allongement, sensiblement parallèle à l'axe du serpentin, et présente une longueur active intégralement contenue entre les spires du serpentin ;
- le serpentin est placé à l'intérieur d'une enceinte thermostatée ;
- le serpentin est positionné en formant un angle compris entre 10° et 15° avec l'horizontale ;
- la sonde ultrasonore comporte une pluralité d'ailettes, en particulier trois ailettes ;
- le séparateur de phases comprend une vis d'Archimède s'étendant principalement suivant un axe longitudinal présentant un diamètre qui augmente longitudinalement de l'amont vers l'aval ;
- la vis d'Archimède est entourée d'un tube filtrant présentant une porosité comprise entre 20 µm et 500 µm ;
- le dispositif comprend une pompe à vide connectée au séparateur de phases ;
- le dispositif comprend une pompe connectée d'une part à la sortie du mélangeur et d'autre part à l'entrée du serpentin ;
- le serpentin est en verre.

L'invention a également pour objet un procédé d'extraction solide/liquide en continu de molécules d'intérêt à partir d'un broyat de matière solide à l'aide d'un dispositif d'extraction selon l'invention, le mélangeur comprenant une cuve délimitant un volume intérieur, le procédé comprenant l'introduction d'un solvant d'extraction et d'un broyat de matière solide dans le volume intérieur délimité par la cuve du mélangeur, le mélange du solvant d'extraction et du broyat de matière solide pour former une suspension homogène, le passage dans le serpentin d'extraction de la suspension, l'irradiation de la suspension par la sonde ultrasonore à travers le serpentin, et le passage de la suspension dans le séparateur de phases, séparant la suspension en une phase liquide comprenant le solvant d'extraction enrichi en molécules d'intérêt et une phase solide comprenant des résidus d'extraction.

D'autres caractéristiques et avantages de l'invention apparaîtront à la lecture de la description qui va suivre, donnée uniquement à titre d'exemple, et faite en se référant aux dessins annexés dans lesquels :
La Figure 1 est une vue schématique d'un dispositif d'extraction selon l'invention comprenant une sonde ultrasonore à irradiation radiale ;
La Figure 2 est une vue schématique d'un serpentin du dispositif d'extraction de la Figure 1 ;
La Figure 3 est une vue schématique de la sonde ultrasonore du dispositif d'extraction de la Figure 1 ; et
La Figure 4 est une vue schématique en perspective éclatée d'un séparateur de phases du dispositif d'extraction de la Figure 1.

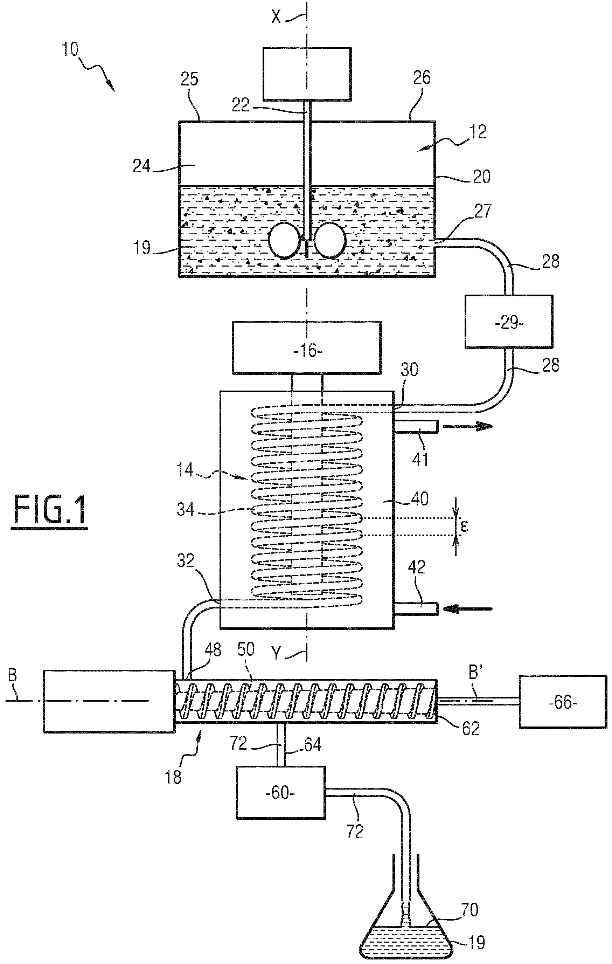

Un dispositif 10 d'extraction solide/liquide est illustré sur la Figure 1.

Le dispositif 10 d'extraction solide/liquide est configuré pour extraire des molécules d'intérêt à partir de matière solide dans un solvant liquide.

En particulier, la matière solide est un broyat d'origine végétale.

A titre d'exemple, le broyat végétal est un broyat de rhizome de Renouée du Japon, et les molécules d'intérêt sont le resvératrol, la polydatine et l'émodine.

Bien entendu, le dispositif 10 d'extraction solide/liquide est adapté à tout autre végétal ou toute autre matière solide susceptible d'être broyée pour en extraire des molécules d'intérêt.

En particulier, le dispositif 10 est adapté à l'éco-extraction et la valorisation de biomasse végétale, par exemple en cosmétique, pharmaceutique, parfumerie, chimie fine et nutraceutique.

En référence à la Figure 1, le dispositif 10 comporte un mélangeur 12, un serpentin 14 d'extraction, une sonde ultrasonore 16 et un séparateur de phases 18.

Dans ce qui suit, les termes « amont » et « aval » et les termes « entrée » et « sortie » sont utilisés en référence aux sens normaux de circulation des fluides dans le dispositif.

Le mélangeur 12 est propre à recevoir la matière solide et le solvant et à former une suspension 19 homogène.

Le mélangeur 12 comprend une cuve 20 et un agitateur mécanique 22 plongé dans la cuve 20.

La cuve 20 délimite un volume intérieur 24.

La cuve 20 s'étend principalement autour d'un axe X et présente de préférence une forme cylindrique à fond plat.

Une telle disposition permet un mélange de la suspension en évitant la sédimentation, c'est-à-dire en évitant le plaquage du broyat végétal au fond de la cuve 20.

En variante, la cuve 20 présente une forme cylindrique et à fond arrondi, ou une forme évasée, tronconique, parallélépipédique, ou toute autre forme envisageable.

La cuve 20 est de préférence réalisée en matière plastique.

En variante, la cuve 20 est réalisée en acier inoxydable résistant aux produits chimiques corrosifs.

Le volume intérieur 24 présente une capacité par exemple comprise entre 1 L et 1000 L, en particulier entre 1 L et 20 L.

Le mélangeur 12 comprend une entrée 25 de solvant et une entrée 26 de matière solide distincte de l'entrée 25 de solvant, et une sortie 27 de suspension 19.

En variante, l'entrée 25 de solvant et l'entrée 26 de matière solide sont confondues.

L'agitateur mécanique 22 est mobile en rotation autour d'un axe X', qui est ici vertical.

De préférence, l'agitateur mécanique 22 et la cuve 20 sont coaxiaux.

L'agitateur mécanique 22 est choisi de façon à ce qu'il plonge au plus profond possible dans le volume intérieur 24 de la cuve 20 pour limiter au maximum la décantation de la matière solide et assurer la meilleure homogénéité de suspension possible.

Par exemple, l'agitateur mécanique 22 est une hélice à pales, ici constitué de trois pales.

La vitesse de rotation de l'agitateur mécanique 22 est adaptée à la viscosité de la suspension. Cette vitesse est généralement comprise entre 60 et 3600 tours par minutes.

La sortie 27 du mélangeur 12 est par exemple l'extrémité d'un tuyau 28 plongé dans la cuve 20.

Le tuyau 28 est par exemple en silicone.

En variante, le tuyau 28 est un tube en acier inoxydable résistant aux produits chimiques corrosifs.

Dans ce qui suit, le terme « diamètre » désigne l'étendue maximale du tuyau ou d'un tube considéré dans un plan transversal, c'est-à-dire perpendiculaire, à l'axe central du tuyau ou du tube. Par exemple, il s'agit du diamètre d'un cercle dans le cas où la section transversale du tuyau ou du tube est circulaire ou la diagonale d'un rectangle dans le cas où la section transversale du tuyau ou du tube est rectangulaire.

Le tuyau 28 a de préférence un diamètre constant sur toute sa longueur.

Par exemple, le tuyau 28 a un diamètre de 8 mm et une longueur de 20 cm à 60 cm.

Le diamètre du tuyau 28 peut bien entendu varier et est adapté aux autres éléments du dispositif 10 et à la matière solide utilisée pour l'extraction.

Il en va de même pour la longueur du tuyau 28.

La sortie 27 du mélangeur 12 est connectée au serpentin 14, par exemple au moyen du tuyau 28.

Avantageusement, le dispositif 10 d'extraction comprend une pompe 29 entre la sortie 27 du mélangeur 12 et le serpentin 14.

La pompe 29 prélève la suspension 19 depuis le volume intérieur 24 de la cuve 20 et l'achemine au serpentin 14.

La pompe 29 est par exemple une pompe péristaltique.

Le réglage de la pompe 29 permet de contrôler le débit de suspension 19 qui entre dans le serpentin 14, et par conséquent la durée d'extraction.

Par exemple, le débit est compris entre 1 L.h⁻¹ et 15 L.h⁻¹, plus particulièrement ici entre 3 L.h⁻¹ et 9 L.h⁻¹.

Le débit ne doit pas être trop élevé pour que le temps de séjour de la suspension 19 dans le serpentin 14 soit suffisant et que l'extraction soit la plus efficace possible.

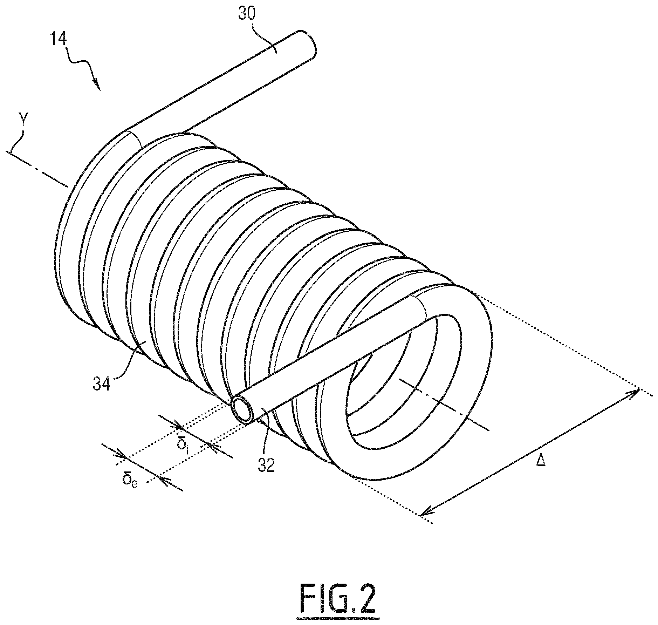

Un exemple de serpentin 14 du dispositif 10 est illustré sur la Figure 2.

L'extraction de molécules d'intérêt issues de la matière solide a lieu dans le serpentin 14.

Le serpentin 14 comprend une entrée 30 et une sortie 32.

L'entrée 30 du serpentin 14 est connectée à la sortie 27 du mélangeur 12.

Le serpentin 14 est formé d'un tube 34 s'enroulant en hélice ou en spirale autour d'un axe Y.

Le tube 34 présente un diamètre intérieur de tube δi, un diamètre extérieur de tube δe.

Le serpentin 14 présente un nombre de spires, un diamètre extérieur de spire Δ mesuré depuis le centre de la spire et jusqu'à l'extérieur de la spire par rapport au centre, et une distance ε entre deux spires consécutives mesurée suivant l'axe Y du serpentin 14 définis.

De préférence, le diamètre intérieur de tube δi, le diamètre extérieur de tube δe, le diamètre extérieur de spire Δ et la distance ε entre deux spires consécutives sont constants sur toute la longueur du serpentin 14 prise suivant l'axe Y du serpentin 14.

Le diamètre extérieur de spire Δ est déterminé de sorte à préserver un espace de réception suffisant pour la sonde ultrasonore 16 mais minimum afin d'en optimiser la puissance ultrasonore appliquée.

En particulier, le tube 34 présente un diamètre intérieur δi compris entre 0,5 cm et 3 cm et notamment égal à 1,4 cm et un diamètre extérieur δe compris entre 0,8 cm et 3,8 cm et notamment égal à 1,8 cm.

En particulier, le serpentin 14 comporte plus de dix spires, notamment treize spires, de diamètre intérieur compris entre 8 cm et 20 cm selon le diamètre de la sonde 16 et notamment égal à 13,5 cm.

La distance ε entre deux spires consécutives est également fixée pour limiter la réflexion des ondes ultrasonores et augmenter l'efficacité de la sonde ultrasonore 16.

En particulier, la distance ε entre deux spires consécutives est compris entre 0,2 cm et 1 cm et notamment égale à 0,3 cm.

Le tube 34 présente par exemple une contenance compris entre 50 mL et 9 L et notamment égale à 880 mL.

L'ajustement du nombre de spires ou l'utilisation de rallonges de serpentin permet de jouer sur la durée d'extraction. En effet, plus le nombre de spires est élevé, dans la limite de la longueur de la sonde ultrasonore 16 prise suivant l'axe Y du serpentin 14 et de la puissance de ladite sonde ultrasonore 16 comme cela sera décrit plus loin, plus la durée d'extraction est élevée, et meilleur est le rendement d'extraction.

Le serpentin 14 est de préférence fabriqué en verre.

Cela permet une réflexion limitée et ainsi une meilleure efficacité des ondes ultrasonores.

Avantageusement, en référence à la Figure 1, le dispositif 10 d'extraction comprend une enceinte thermostatée 40 à l'intérieur de laquelle est placé le serpentin 14.

Cela permet de contrôler la température d'extraction selon les molécules à extraire.

Selon un premier mode de réalisation, le serpentin 14 est positionné verticalement dans l'enceinte thermostatée 40, comme c'est le cas sur la Figure 1, de sorte que l'entrée 30 du serpentin 14 soit placée à une hauteur supérieure à celle de la sortie 32, et que la suspension 19 parcoure le serpentin 14 poussée par la gravité.

L'encombrement au sol du dispositif 10 est ainsi limité.

En variante, le serpentin 14 est positionné pour former un angle compris entre 10° et 15° par rapport à l'horizontale.

Ceci permet de contrôler au mieux le temps de séjour de la suspension 19 dans le serpentin 14 en limitant les effets dus à la gravité.

L'enceinte thermostatée 40 est typiquement un récipient recevant un fluide caloporteur et couplé à un système de régulation de la température.

Par exemple, le fluide caloporteur est un mélange d'eau et d'éthylène-glycol, chacun à 50% en volume du volume total de fluide caloporteur.

Le système de régulation de la température est par exemple assuré par un compresseur permettant de régler la température du fluide caloporteur et de la maintenir sensiblement constante.

Selon la matière solide et les molécules d'intérêt à extraire, la température est réglée entre 5 °C et 75 °C.

L'enceinte thermostatée 40 comprend une entrée 41 et une sortie 42 de fluide caloporteur.

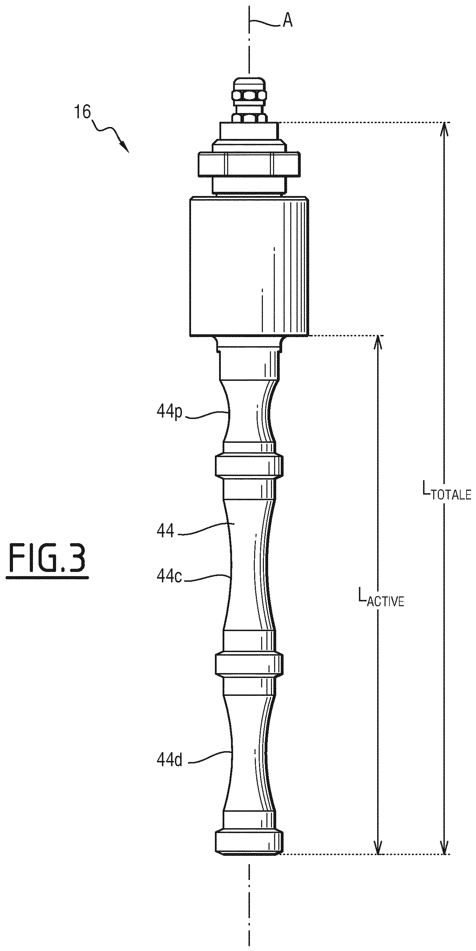

L'extraction est assistée par la sonde ultrasonore 16 à émission radiale illustrée sur la Figure 3.

La sonde ultrasonore 16 est plongée dans l'enceinte thermostatée 40.

La sonde ultrasonore 16 est positionnée au centre du serpentin 14.

La sonde ultrasonore 16 s'étend principalement suivant une direction d'allongement A.

De préférence, la direction d'allongement A est sensiblement parallèle à l'axe Y du serpentin 14 lorsque la sonde ultrasonore 16 est en place.

La sonde ultrasonore 16 est par exemple à une distance radiale maximale inférieure à 5 cm selon le diamètre de la sonde, notamment de 3 cm de chaque spire du serpentin 14.

Comme visible sur la Figure 3, la sonde ultrasonore 16 présente une longueur totale Lₜₒₜₐₗₑ mesurée suivant la direction d'allongement A comprise entre 25 cm et 35 cm.

La sonde ultrasonore 16 présente une longueur active L_{active} comprise entre 20 cm et 30 cm.

Par longueur active L_{active}, il est entendu la longueur de la sonde ultrasonore 16 sur laquelle la sonde ultrasonore 16 est capable d'émettre des ultrasons.

En particulier la sonde ultrasonore 16 présente une longueur active L_{active} égale à 24,2 cm.

La longueur active L_{active} de la sonde ultrasonore 16 est avantageusement intégralement contenue entre les spires du serpentin 14 et de manière coaxiale par rapport au serpentin 14.

La sonde ultrasonore 16 comporte une pluralité d'ailettes 44.

Par ailette, il est entendu une tige s'étendant entre deux extrémités et présentant une forme évasée en direction de chacune des deux extrémités.

En particulier, la sonde ultrasonore 16 comporte trois ailettes 44, une ailette proximale 44p, une ailette centrale 44c et une ailette distale 44d de l'amont vers l'aval du serpentin 14.

Chaque ailette 44 présente par exemple un diamètre maximal compris entre 1 cm et 10 cm.

En particulier, chaque ailette 44 présente un diamètre maximal égal à 3 cm, et un diamètre minimal égal à 1,6 cm.

L'ailette centrale 44c et l'ailette distale 44d sont identiques.

L'ailette proximale 44p présente une longueur inférieure à la longueur de l'ailette centrale 44c ou de l'ailette distale 44d mesurée suivant la direction d'allongement A de la sonde ultrasonore 16.

La puissance électrique de la sonde ultrasonore 16 est comprise entre 75 W et 750 W.

Le réglage de la puissance électrique permet d'optimiser l'efficacité d'extraction des différentes molécules ciblées.

La fréquence de la sonde ultrasonore 16 est comprise entre 20 et 80 kHz.

L'irradiation ultrasonore en continu permet d'augmenter le transfert des molécules d'intérêt de la matière solide vers le solvant, en fonction du temps de séjour dans le serpentin 14 et de la puissance acoustique, tout en maintenant la fluidité et l'homogénéité de la suspension 19.

Le positionnement de la sonde ultrasonore 16 au centre du serpentin 14 est tel qu'aucun contact direct n'est possible entre la sonde ultrasonore et la matière solide en suspension dans le solvant d'extraction, limitant ainsi tout risque de contamination de la suspension.

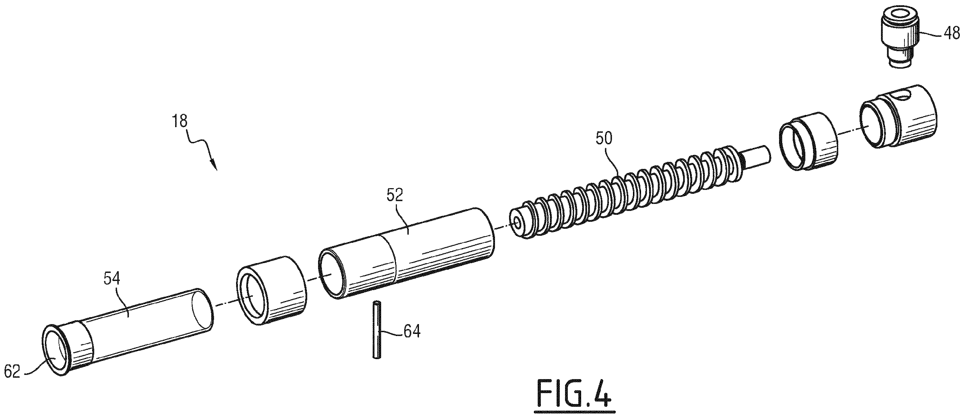

La sortie 32 du serpentin 14 est connectée au séparateur de phases 18 illustré sur la Figure 4.

Le séparateur de phases 18 comprend une entrée 48 par laquelle entre la suspension 19 après son passage dans le serpentin 14.

Le séparateur de phases 18 comprend par exemple une vis d'Archimède 50.

La vis d'Archimède 50 s'étend principalement suivant un axe longitudinal B-B'.

L'axe B-B' de la vis d'Archimède 50 présente un diamètre qui augmente avantageusement de l'amont vers l'aval.

Ceci permet de compacter la suspension au fur et à mesure de son passage dans la vis d'Archimède 50 et ainsi de récupérer un maximum de solvant enrichi en molécules d'intérêt extraites de la matière solide.

La vis d'Archimède 50 présente un diamètre maximal par exemple compris entre 1 cm et 50 cm.

En particulier, la vis d'Archimède 50 présente un diamètre maximal égal à 25 mm.

La vis d'Archimède 50 est d'une longueur, mesurée suivant son axe B-B', comprise par exemple entre 10 cm et 200 cm.

En particulier, la vis d'Archimède 50 est d'une longueur égale à 15 cm.

La vis d'Archimède 50 est avantageusement entourée d'un tube 52 filtrant.

Le tube 52 est par exemple constitué d'une toile métallique en acier inoxydable.

Le tube 52 présente une porosité comprise entre 20 µm et 500 µm.

En particulier, le tube 52 présente une porosité comprise sensiblement égale à 50 µm.

La vis d'Archimède 50 et le tube 52 sont avantageusement placés dans une enveloppe tubulaire 54.

L'enveloppe tubulaire 54 est par exemple en matière plexiglas.

L'enveloppe tubulaire 54 présente par exemple un diamètre compris entre 2,5 cm et 50 cm.

En particulier, l'enveloppe tubulaire 54 présente un diamètre égal à 3,5 cm.

L'enveloppe tubulaire 54 présente une longueur mesurée suivant l'axe B-B' de la vis d'Archimède 50, comprise par exemple entre 10 cm et 200 cm.

En particulier, l'enveloppe tubulaire 54 présente une longueur égale à 15 cm.

La vis d'Archimède 50 est avantageusement actionnée par un moteur électrique commandé par un variateur de vitesse compris entre 1 et 100 tours par minute.

Le moteur présente par exemple une puissance maximale tolérée de 15 V et 1 A.

L'humidité du solide récupéré en sortie est dépendante de la vitesse de rotation de la vis d'Archimède 50. Si la vis d'Archimède 50 tourne trop rapidement, la matière n'est pas essorée suffisamment longtemps avant d'être expulsée en sortie.

En référence à la Figure 1, le dispositif 10 comprend avantageusement une pompe à vide 60 connectée au séparateur de phases 18.

La pompe à vide 60 est par exemple une pompe péristaltique.

La pompe à vide 60 est de préférence une pompe à membrane équipée d'un manomètre électronique.

La pompe à vide 60 est par exemple configurée pour que la pression mesurée dans la vis d'Archimède 50 soit comprise entre 100 mbar et 900 mbar.

En particulier, la pompe à vide 60 est configurée pour que la pression mesurée dans la vis d'Archimède 50 soit égale à 800 mbar.

Un vide trop poussé n'est pas souhaitable, car cela boucherait la vis d'Archimède 50.

Le séparateur de phases 18 comprend une sortie 62 de phase solide en aval de la vis d'Archimède 50 et une sortie 64 de phase liquide comprenant le solvant enrichi en molécules d'intérêt.

La sortie 64 de phase liquide est avantageusement diamétralement opposée à l'entrée 48 du séparateur de phases 18.

L'agencement du séparateur de phases 18 avec la pompe à vide 60 permet de récupérer un maximum de solvant enrichi sans matière solide et d'éliminer un gâteau de résidu d'extraction solide quasiment sec qui peut être valorisé ensuite.

Le séparateur de phases 18 permet l'entrée simultanée des phases solide et liquide. Les phénomènes de refoulement observés avec des systèmes commerciaux à contre-courant sont ainsi évités.

Le dispositif 10 comprend avantageusement un bac 66 de réception de phase solide au voisinage de la sortie 62 du séparateur de phases 18.

Le dispositif 10 comprend avantageusement en outre un décanteur 70 relié à la sortie 64 de phase liquide.

Le décanteur 70 permet de séparer par gravité les particules fines restant dans la phase liquide.

Par exemple, le décanteur 70 est un erlenmeyer.

Le solvant enrichi en molécules d'intérêt obtenu en sortie du séparateur de phases 18 est par exemple acheminé *via* la pompe à vide 60 jusqu'au décanteur 70.

Le décanteur 70 est par exemple relié à la sortie 64 de solvant enrichi en molécules d'intérêt par un tube 72.

Le tube 72 est par exemple réalisé en matière silicone.

La pompe à vide 60 a le double avantage de créer un vide et d'acheminer la phase liquide jusqu'au décanteur 70. Pour que le vide de la pompe à vide 60 soit efficace, le tube 72 doit être le plus rigide possible.

Le séparateur de phases 18 et la pompe à vide 60 permettent l'obtention très rapide d'une solution liquide limpide, typiquement en moins d'une heure.

Le dispositif 10 selon l'invention permet d'extraire et de séparer une phase liquide contenant le solvant enrichi en molécules d'intérêt de la phase solide, de façon continue, à partir de broyat de matière solide.

La sonde ultrasonore 16 à irradiation radiale n'est pas en contact direct avec la suspension 19, limitant tout risque de contamination. Le réglage de la puissance acoustique permet d'optimiser l'efficacité d'extraction des différentes molécules d'intérêt ciblées.

Le dispositif présente également l'avantage d'être compact, mobile et utilisable à des débits importants.

En outre, de nombreux paramètres sont modulables en fonction des molécules d'intérêt à extraire : le ratio solide/liquide de la suspension 19 par le réglage de la vitesse de rotation de la vis d'Archimède 50 apportant la matière solide dans le mélangeur 12, et du débit de la pompe entraînant le solvant dans le mélangeur 12, la température d'extraction par le réglage de la température de l'enceinte thermostatée 40, la durée d'extraction par le réglage du débit de la pompe 29 entraînant la suspension 19 vers l'entrée 30 du serpentin 14 ou par la longueur du serpentin 14.

Le dispositif 10 permet de limiter les consommations en solvants et en énergie, et ne nécessite pas de consommables pour fonctionner, tels que des filtres, et donc permet de limiter les coûts d'utilisation.

Un procédé d'extraction de molécules d'intérêt à partir de matière solide à l'aide du dispositif 10 d'extraction va maintenant être décrit.

Un solvant d'extraction et un broyat de matière solide sont introduits dans le volume intérieur 24 délimité par la cuve 20 du mélangeur 12.

Le solvant d'extraction est avantageusement un mélange d'eau et d'éthanol.

En variante, le solvant d'extraction est un solvant ou un mélange de solvants de nature différente, techniquement envisageable pour réaliser une extraction solide/liquide.

Les proportions du mélange d'eau et d'éthanol sont comprises entre 1:99 et 99:1 en volume par rapport au volume total de solvant.

Le solvant d'extraction est par exemple préparé en amont puis acheminé jusqu'au volume intérieur 24 de la cuve 20 par une pompe doseuse.

En variante, le solvant d'extraction est réalisé directement en entrée du mélangeur 20 grâce à une pompe binaire.

La matière solide est par exemple apportée sous forme de poudre sèche à une granulométrie comprise entre 0,2 mm et 1 mm.

La matière solide est par exemple apportée au moyen d'une vis d'Archimède de vitesse de rotation fixée pour maintenir une concentration fixe dans le mélangeur 12.

Le solvant d'extraction et la matière solide sont alimentés en continu dans le mélangeur 12 pour former une suspension 19.

Le réglage de la vitesse de rotation de la vis d'Archimède et du débit de la pompe du solvant en entrée du mélangeur 12 permet de faire varier le ratio solide/liquide de la suspension 19 selon le besoin.

Une suspension 19 homogène est obtenue. Par exemple, la concentration massique de broyat végétal dans le solvant d'extraction est comprise entre 100 g.L⁻¹ et 150 g.L⁻¹.

La suspension 19 est entraînée de la sortie 27 du mélangeur 12 à l'entrée 30 du serpentin 14.

Avantageusement, la pompe 29 fixe le débit d'entraînement de la suspension 19.

Par exemple, le débit est compris entre 1 L.h⁻¹ et 15 L.h⁻¹, plus particulièrement entre 3 L.h⁻¹ et 9 L.h⁻¹.

Le débit est réglé de manière à contrôler le temps de séjour moyen de la suspension 19 dans le serpentin 14 pour atteindre un rendement d'extraction maximal.

Avantageusement, le temps de séjour de la suspension dans le serpentin 14 est compris entre 30 secondes et 30 minutes, de préférence entre 5 minutes et 30 minutes.

L'enceinte thermostatée 40 est réglée à une température fixe afin de contrôler la température d'extraction. Par exemple, la température de l'enceinte thermostatée 40 est comprise entre 5°C et 75 °C.

La sonde ultrasonore 16 est connectée à un générateur lui permettant d'irradier le contenu du serpentin 14 à une fréquence comprise entre 20 kHz et 80 kHz, en particulier à 20 kHz.

Sous l'effet de la température et de l'irradiation ultrasonore, les molécules d'intérêt sont extraites de la matière solide par le solvant d'extraction.

La suspension 19 dans le solvant enrichi en molécules d'intérêt est acheminée de la sortie 32 du serpentin 14 au séparateur de phases 18.

Le débit d'entrée de la suspension dans le séparateur de phases 18 est égal au débit d'entraînement de la suspension du mélangeur 12 au serpentin 14.

La séparation de phases est de préférence réalisée par pressage sous vide au travers de la vis d'Archimède 50, poussant la phase solide essorée vers l'extérieur de la vis d'Archimède 50.

La phase liquide comprenant le solvant enrichi est évacuée plus efficacement grâce à l'aspiration sous vide assurée par la pompe à vide 60.

La pompe à vide 60 est par exemple réglée pour que la pression mesurée dans la vis d'Archimède 50 soit comprise entre 100 mbar et 900 mbar.

En particulier, la pompe à vide 60 est réglée pour que la pression mesurée dans la vis d'Archimède 50 soit égale à 800 mbar.

La phase liquide ayant traversé le tube 52 entourant la vis d'Archimède 50 est récupérée dans le décanteur 70.

Grâce au procédé décrit selon l'invention, le solvant enrichi en molécules d'intérêt est récupéré totalement exempt de particules solides de granulométrie supérieure à 20 µm.

Si nécessaire, l'ajout d'une cartouche papier permet d'éliminer les particules solides de granulométrie supérieure à 5 µm.

Cette étape de filtration permet l'utilisation directe du solvant enrichi en molécules d'intérêt dans un procédé de purification par exemple.

Les rendements d'extraction globaux sont améliorés grâce à la combinaison du pressage par vis d'Archimède et de l'aspiration sous vide pour récolter le maximum de solvant enrichi en molécules d'intérêt.

En outre, la phase solide est récupérée essorée et séparée du solvant et reste ainsi disponible pour être valorisée.

Le procédé décrit dans l'invention permet une extraction, une filtration et une séparation simplifiées par l'utilisation d'un seul dispositif.

Après utilisation, le nettoyage du dispositif 10 est avantageusement aisé.

Un liquide de nettoyage est prélevé dans la cuve 20 du mélangeur 12 et acheminé dans le serpentin 14 en maintenant l'irradiation ultrasonore le temps que l'intégralité de la matière solide soit évacuée du serpentin 14.

Par exemple, le liquide de nettoyage est de l'eau déminéralisée.

Pour nettoyer le séparateur de phases 18 des particules fines qui auraient pu se déposer sur le tube 52 entourant la vis d'Archimède 50, un liquide de nettoyage est acheminé dans les conditions de l'extraction, en maintenant la rotation de la vis d'Archimède et l'aspiration par la pompe à vide 60.

Par exemple, le liquide de nettoyage est de l'eau déminéralisée.

Pour nettoyer la vis d'Archimède 50 des résidus de solide, un liquide de nettoyage est acheminé en maintenant la rotation de la vis d'Archimède et en coupant l'aspiration par la pompe à vide 60.

Par exemple, le liquide de nettoyage est de l'eau déminéralisée.

Le procédé est ainsi adapté à tout type d'extraction solide/liquide mettant en jeu une matière solide dont la granulométrie est comprise entre 0,2 mm et 1 mm, et un solvant d'extraction dans le but d'enrichir le solvant en molécules d'intérêt et le séparer, en continu, de la phase solide.

### Exemple de mise en oeuvre du dispositif selon l'invention

### Conditions opératoires :

- Solvant EtOH/H₂O (6/4 v/v).
- Granulométrie du broyat végétal 200 µm < G < 1 mm.
- concentration massique de broyat végétal en suspension dans le solvant : 100 g.L-1
- Débit de la pompe péristaltique entre le mélangeur et le serpentin : 50 mL.min⁻¹, 3 L.h⁻¹.
- Amplitude réglée sur le générateur à ultrasons : 100 % de la puissance électrique maximale (750 W).
- Vis d'Archimède : 13 V, 0.8 A.

### 1- Cuve contenant la suspension

La suspension est préparée avec du rhizome de Renouée du Japon broyé à une granulométrie comprise entre 200 µm et 1 mm. La granulométrie est contrôlée grâce à des tamis en inox utilisés sur la poudre sèche.

Si la poudre contient des particules de granulométrie inférieure à 200 µm, le système de filtration se bouche très rapidement.

### 2- Agitateur mécanique

L'agitateur mécanique est réglé à une vitesse de rotation de 500 tours par minute. L'hélice constituée de trois pales plonge au plus profond possible dans la cuve pour limiter au maximum la décantation de la matière solide et assurer la meilleure homogénéité possible.

### 3- Pompe péristaltique

La pompe péristaltique est réglée pour un débit minimum de 50 mL.min⁻¹ (3 L.h⁻¹), soit un temps de séjour compris entre 60 secondes et 90 secondes dans le serpentin dans le cadre des tests réalisés. En dessous de cette vitesse, il existe un risque d'agglomération de la suspension et bouchage dans le tuyau qui apporte la suspension du mélangeur au serpentin. Le tuyau a un diamètre de 8 mm.

### 4- Enceinte thermostatée

La température de l'enceinte est contrôlée à l'aide d'un refroidisseur à circulation de type MinichHiller^{®} de Huber par exemple, ou d'un chauffage à bain d'huile.

### 5- Sonde ultrasonore radiale

La sonde ultrasonore radiale est positionnée au centre du serpentin en verre et réglée à une fréquence de 20 kHz. La longueur active de la sonde ultrasonore est intégralement contenue au sein des spires du serpentin de manière coaxiale au serpentin.

La sonde est éloignée de 3 cm de chaque côté du serpentin. La puissance maximale est de 750 W et peut être réglée de 10 à 100 % de sa puissance nominale. La sonde ultrasonore radiale et le générateur associé sont de marque SinapTec par exemple (Ultrasonic Processor NextGen Lab750).

Lors des tests, la sonde a été utilisée au maximum de sa capacité sans problème notoire ni au niveau du serpentin ni au niveau de l'enceinte thermostatée.

### 6- Serpentin

Le serpentin en verre est constitué de 13 spires de 13,5 cm de diamètre extérieur. Le tube du serpentin a un diamètre intérieur de 1,4 cm et une longueur totale de 5,7 m, soit une contenance de 880 mL. Le serpentin est placé verticalement, l'entrée étant placée plus haut que la sortie, de sorte que la suspension s'écoule par gravité.

### 7- Vis d'Archimède pour la séparation solide/liquide

La suspension récoltée en sortie du serpentin est séparée continuellement en deux phases : liquide et solide. La suspension entre verticalement dans le système de filtration continue. La phase liquide est extraite sous vide à partir d'un tube diamétralement opposé à l'entrée de la suspension. La vis d'Archimède entourée d'une grille tubulaire en acier inoxydable de porosité 50 µm permet d'acheminer la phase solide desséchée au fur et à mesure que la phase liquide est aspirée. Le moteur actionnant la vis d'Archimède est alimenté par un courant de 13 V et 0,8 A. La puissance admise par le moteur est directement réglée sur le boitier d'alimentation. L'humidité du solide récupéré en sortie est dépendante de la vitesse de rotation de la vis. Si la vis tourne trop rapidement, la matière n'est pas suffisamment essorée avant d'être expulsée en sortie. Dans les conditions des tests, un réglage sur 13 V a permis une vitesse optimale pour un débit de 3 L.h⁻¹. En dessous de 13 V, le solide peut se compacter dans la vis et à boucher le dispositif.

Les valeurs données ici sont à titre indicatif, un changement de débit de pompe ou des paramètres de dilution et de granulométrie impacteraient directement ces valeurs.

### 8- Récupération du solide

La phase solide quasiment sèche est récupérée dans un bac en sortie de vis.

### 9- Décanteur - récupération de la phase liquide

La phase liquide obtenue en sortie du séparateur de phases est acheminée *via* une pompe péristaltique jusqu'à un erlenmeyer permettant de décanter les particules fines dont la granulométrie est inférieure à 50 µm.

### 10- Pompe péristaltique pour la récupération de la phase liquide.

Afin d'assurer un meilleur essorage du solide, le séparateur de phases est placé sous vide. Ce vide est assuré par la pompe péristaltique mentionnée ci-dessus qui a le double avantage de créer un vide et d'acheminer la phase liquide jusqu'au décanteur.

## Revendications

1. Dispositif (10) d'extraction solide/liquide en continu comprenant au moins, d'amont en aval :
un mélangeur (12) comprenant une entrée (26) et une sortie (27), un serpentin (14) d'extraction comprenant une entrée (30) et une sortie (32), l'entrée (30) du serpentin (14) étant connectée à la sortie du mélangeur (12), et un séparateur de phases (18) connecté à la sortie du serpentin (14),
**caractérisé en ce qu'**il comprend une sonde ultrasonore (16) à irradiation radiale placée au centre du serpentin (14).

2. Dispositif (10) selon la revendication 1, dans lequel le serpentin (14) est formé d'un tube (34) s'enroulant en hélice autour d'un axe (Y) pour former des spires, et dans lequel la sonde ultrasonore (16) s'étend principalement suivant une direction d'allongement (A), sensiblement parallèle à l'axe (Y) du serpentin (14), et présente une longueur active (L_{active}) intégralement contenue entre les spires du serpentin (14).

3. Dispositif (10) selon la revendication 1 ou 2, dans lequel le serpentin (14) est placé à l'intérieur d'une enceinte thermostatée (40).

4. Dispositif (10) selon l'une quelconque des revendications 1 à 3, dans lequel le serpentin (14) est positionné en formant un angle compris entre 10° et 15° avec l'horizontale.

5. Dispositif (10) selon l'une quelconque des revendications 1 à 4, dans lequel la sonde ultrasonore (16) comporte une pluralité d'ailettes, en particulier trois ailettes (44).

6. Dispositif (10) selon l'une quelconque des revendications 1 à 5, dans lequel le séparateur de phases (18) comprend une vis d'Archimède (50) s'étendant principalement suivant un axe longitudinal (B-B') présentant un diamètre qui augmente longitudinalement de l'amont vers l'aval.

7. Dispositif (10) selon la revendication 6, dans lequel la vis d'Archimède (50) est entourée d'un tube (52) filtrant présentant une porosité comprise entre 20 µm et 500 µm.

8. Dispositif (10) selon l'une quelconque des revendications 1 à 7, comprenant une pompe à vide (60) connectée au séparateur de phases (18).

9. Dispositif (10) selon l'une quelconque des revendications 1 à 8, comprenant une pompe (29) connectée d'une part à la sortie (27) du mélangeur (12) et d'autre part à l'entrée du serpentin (14).

10. Dispositif (10) selon l'une quelconque des revendications 1 à 9, dans lequel le serpentin (14) est en verre.

11. Procédé d'extraction solide/liquide en continu de molécules d'intérêt à partir d'un broyat de matière solide à l'aide d'un dispositif d'extraction (10) selon l'une quelconque des revendications 1 à 10, le mélangeur (12) comprenant une cuve (20) délimitant un volume intérieur (24), le procédé comprenant l'introduction d'un solvant d'extraction et d'un broyat de matière solide dans le volume intérieur (24) délimité par la cuve (20) du mélangeur (12), le mélange du solvant d'extraction et du broyat de matière solide pour former une suspension (19) homogène, le passage dans le serpentin (14) d'extraction de la suspension (19), l'irradiation de la suspension (19) par la sonde ultrasonore (16) à travers le serpentin (14), et le passage de la suspension (19) dans le séparateur de phases (18), séparant la suspension (19) en une phase liquide comprenant le solvant d'extraction enrichi en molécules d'intérêt et une phase solide comprenant des résidus d'extraction.

## Patentansprüche

1. Vorrichtung (10) zur kontinuierlichen Fest-/Flüssigextraktion, mindestens umfassend von stromaufwärts nach stromabwärts:
einen Mischer (12), umfassend einen Einlass (26) und einen Auslass (27), eine Extraktionsrohrschlange (14), umfassend einen Einlass (30) und einen Auslass (32), wobei der Einlass (30) der Rohrschlange (14) mit dem Auslass des Mischers (12) verbunden ist, und einen Phasentrenner (18), der mit dem Auslass der Rohrschlange (14) verbunden ist,
**dadurch gekennzeichnet, dass** sie eine Ultraschallsonde (16) mit radialer Bestrahlung umfasst, die in der Mitte der Rohrschlange (14) platziert ist.

2. Vorrichtung (10) nach Anspruch 1, wobei die Rohrschlange (14) aus einem Rohr (34) gebildet ist, das sich schraubenförmig um eine Achse (Y) windet, um Windungen zu bilden, und wobei sich die Ultraschallsonde (16) hauptsächlich entlang einer Längsrichtung (A) im Wesentlichen parallel zu der Achse (Y) der Spule (14) erstreckt, und eine aktive Länge (Lₐₖₜᵢᵥ) aufweist, die vollständig zwischen den Windungen der Rohrschlange (14) enthalten ist.

3. Vorrichtung (10) nach Anspruch 1 oder 2, wobei die Rohrschlange (14) innerhalb eines thermostatisierten Gehäuses (40) platziert ist.

4. Vorrichtung (10) nach einem der Ansprüche 1 bis 3, wobei die Rohrschlange (14) positioniert ist, um einen Winkel zwischen 10° und 15° zur Horizontalen zu bilden.

5. Vorrichtung (10) nach einem der Ansprüche 1 bis 4, wobei die Ultraschallsonde (16) eine Vielzahl von Rippen, insbesondere drei Rippen (44), umfasst.

6. Vorrichtung (10) nach einem der Ansprüche 1 bis 5, wobei der Phasentrenner (18) eine archimedische Schraube (50) umfasst, die sich hauptsächlich entlang einer Längsachse (B-B') erstreckt und einen Durchmesser aufweist, der in Längsrichtung von stromaufwärts nach stromabwärts zunimmt.

7. Vorrichtung (10) nach Anspruch 6, wobei die archimedische Schraube (50) von einem Filterrohr (52) umgeben ist, das eine Porosität zwischen 20 µm und 500 µm aufweist.

8. Vorrichtung (10) nach einem der Ansprüche 1 bis 7, umfassend eine Vakuumpumpe (60), die mit dem Phasentrenner (18) verbunden ist.

9. Vorrichtung (10) nach einem der Ansprüche 1 bis 8, umfassend eine Pumpe (29), die einerseits mit dem Auslass (27) des Mischers (12) und andererseits mit dem Einlass der Rohrschlange (14) verbunden ist.

10. Vorrichtung (10) nach einem der Ansprüche 1 bis 9, wobei die Rohrschlange (14) aus Glas ist.

11. Verfahren zur kontinuierlichen Fest-/Flüssigextraktion von Molekülen von Interesse aus einem Feststoffmahlgut mittels einer Extraktionsvorrichtung (10) nach einem der Ansprüche 1 bis 10, der Mischer (12) umfassend einen Behälter (20), der ein Innenvolumen (24) begrenzt, das Verfahren umfassend das Einführen eines Extraktionslösungsmittels und eines Mahlguts aus festem Material in das Innenvolumen (24), das durch den Behälter (20) des Mischers (12) begrenzt wird, das Mischen des Extraktionslösungsmittels und des Mahlguts aus festem Material, um eine homogene Suspension (19) zu bilden, den Durchgang durch die Extraktionsrohrschlange (14) der Suspension (19), die Bestrahlung der Suspension (19) durch die Ultraschallsonde (16) durch die Rohrschlange (14), und den Durchgang der Suspension (19) durch den Phasentrenner (18), wobei die Suspension (19) in eine flüssige Phase, die das mit den Molekülen von Interesse angereicherte Extraktionslösungsmittel umfasst, und eine feste Phase, die Extraktionsrückstände umfasst, getrennt wird.

## Claims

1. A device (10) for continuous solid/liquid extraction comprising at least, from upstream to downstream:
a mixer (12) comprising an inlet (26) and an outlet (27), an extraction coil (14) comprising an inlet (30) and an outlet (32), the inlet (30) of the coil (14) being connected to the outlet of the mixer (12), and a phase separator (18) connected to the outlet of the coil (14),
**characterized in that** it comprises an ultrasonic probe (16) with radial irradiation placed in the center of the coil (14).

2. The device (10) depending on claim 1, wherein the coil (14) is formed of a tube (34) extending helically about an axis (Y) so as to form turns, and wherein the ultrasonic probe (16) extends mainly along an elongation direction (A), substantially parallel to the axis (Y) of the coil (14) and has an active length (L_{active}) entirely contained between the turns of the coil (14).

3. The device (10) depending on claim 1 or 2, wherein the coil (14) is located within a thermostatically controlled chamber (40).

4. The device (10) depending on any of claims 1 to 3, wherein the coil (14) is positioned by forming an angle comprised between 10° and 15° with the horizontal.

5. The system (10) depending on any of claims 1 to 4, wherein the ultrasonic probe (16) has a plurality of fins, in particular three fins (44).

6. The system (10) depending on any of claims 1 to 5, wherein the phase separator (18) comprises an Archimedes screw (50) extending mainly along a longitudinal axis (B-B') having a diameter which increases longitudinally from upstream to downstream.

7. The system (10) depending on claim 6, wherein the Archimedes screw (50) is surrounded by a filter tube (52) having a porosity comprised between 20 µm and 500 µm.

8. The system (10) depending on any of claims 1 to 7, comprising a vacuum pump (60) connected to the phase separator (18).

9. The system (10) depending on any of claims 1 to 8, comprising a pump (29) connected on the one hand to the outlet (27) of the mixer (12) and on the other hand to the inlet of the coil (14).

10. The system (10) depending on any of claims 1 to 9, wherein the coil (14) is made of glass.

11. A method of continuous solid/liquid extraction of molecules of interest from a ground solid material by using an extraction system (10) depending on any of claims 1 to 10, the mixer (12) comprising a tank (20) delimiting an internal volume (24), the method comprising introducing an extraction solvent and a solid ground material into the internal volume (24) delimited by the tank (20) of the mixer (12), mixing the extraction solvent and the ground solid material so as to form a homogeneous suspension (19), passing through the coil (14) for extracting the suspension (19), irradiating the suspension (19) by means of the ultrasonic probe (16) through the coil (14), and passing the suspension (19) through the phase separator (18), separating the suspension (19) into a liquid phase comprising the extraction solvent enriched with molecules of interest and a solid phase comprising extraction residues.
